(19) Europäisches Patentamt
European Patent Office
Office européen des brevets

(11) Veröffentlichungsnummer: **0 229 593 B1**

# (12) EUROPÄISCHE PATENTSCHRIFT

(45) Veröffentlichungstag der Patentschrift: **16.10.91**

(51) Int. Cl.[5]: **C07C 45/41**, C07C 47/548, C07C 65/30, C07C 51/377

(21) Anmeldenummer: **86810587.5**

(22) Anmeldetag: **15.12.86**

## (54) Verfahren zur Herstellung von 4,4'-Stilbendialdehyden.

(30) Priorität: **19.12.85 CH 5447/85**

(43) Veröffentlichungstag der Anmeldung:
**22.07.87 Patentblatt 87/30**

(45) Bekanntmachung des Hinweises auf die Patenterteilung:
**16.10.91 Patentblatt 91/42**

(84) Benannte Vertragsstaaten:
**CH DE ES FR GB IT LI**

(56) Entgegenhaltungen:
**EP-A- 0 008 123**
**AT-B- 367 012**
**DE-A- 1 914 112**
**EP-A- 0 104 296**
**DE-A- 1 643 639**
**DE-A- 3 129 273**

(73) Patentinhaber: **CIBA-GEIGY AG**
**Klybeckstrasse 141**
**CH-4002 Basel(CH)**

(72) Erfinder: **Reinehr, Dieter, Dr.**
**Wolfsheule 10**
**W-7842 Kandern(DE)**
Erfinder: **Steiner, Heinz**
**Riehenring 9/1**
**CH-4058 Basel(CH)**

**EP 0 229 593 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Herstellung von Aldehyden aus Carbonsäurechloriden ist als Rosenmund-Reduktion schon lange bekannt. Eine allgemeine Übertragbarkeit des Verfahrens scheiterte jedoch an den Reaktionsbedingungen, die häufig eine Weitereaktion bis hin zum Alkan ermöglichen. In seinen Untersuchungen zum Einfluss der Reaktionstemperatur kommen D.P. Wagner et al. zum Ergebnis, dass erhöhte Temperaturen eine starke Ausbeuteerniedrigung zur Folge haben (Ann. N.Y. Acad. Sci. Vol. 172, S186 ff (1970)). Zur Herstellung spezieller Klassen von Aldehyden wird in der DOS 31 29 273 die Reduktion von Arylsäurehalogeniden mit Wasserstoff in einem ausgewählten Lösungsmittel (Sulfon) in Gegenwart eies $PdBaSO_4$-Katalysators beschrieben. In der DOS 19 14 112 wird die Durchführung der Reduktion von Carbonsäurehalogeniden zu aromatischen bzw. heterocyclischen Aldehyden mit Wasserstoff, wiederum in Gegenwart eines $PdBaSO_4$-Katalysators, der gegebenenfalls mit Schwefelverbindungen vergiftet ist, in inerten organischen Lösungsmitteln wie z.B. Xylol oder Toluol, vorgeschlagen.

Die vorliegende Erfindung betrifft ein neues Verfahren zur Herstellung von 4,4'-Stilbendialdehyden der Formel

(1) 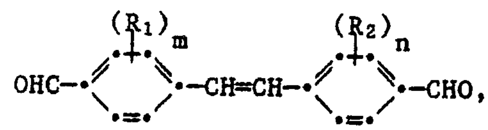

worin $R_1$ und $R_2$ unabhängig voneinander Substituenten sind, die unter den unten definierten Bedingungen nicht reduziert werden können, und m und n unabhängig voneinander 0, 1 oder 2 sind.

Das erfindungsgemässe Verfahren ist dadurch gekennzeichnet, dass man Verbindungen der Formel

(2) 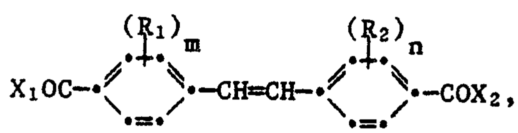

worin $R_1$, $R_2$, m und n die angegebenen Bedeutungen haben und $X_1$ und $X_2$ unabhängig voneinander Wasserstoff, Chlor oder Brom sind, wobei $X_1$ und $X_2$ nicht gleichzeitig Wasserstoff sind, in einem wasserfreien, inerten, aprotischen, organischen Lösungsmittel in Gegenwart eines Edelmetallkatalysators bei einer Temperatur von 15 bis 200 °C und einem Druck von 1 bis 50 bar so lange hydriert, bis eine zuvor berechnete Menge Wasserstoff, die theoretisch nötig ist, um die Verbindungen der Formel (1) zu erhalten, aufgenommen worden ist, den Katalysator abtrennt und die Verbindung isoliert.

Die Verbindungen der Formel (2) sind teilweise bekannt ($X_1$ und $X_2$ gleichzeitig Wasserstoff, Chlor und Brom) und teilweise neu ($X_1$ Wasserstoff und $X_2$ Chlor oder Brom).

Gegenstand der vorliegenden Erfindung sind des weiteren somit auch die neuen Verbindungen der Formel (2), und ein Verfahren zur Herstellung dieser Verbindungen. Die Verwendung der Verbindungen der Formel (2) zur Herstellung von 4,4'-Stilbendialdehyden ist ein weiterer Gegenstand der Erfindung.

In den Verbindungen der Formel (1) bedeuten $R_1$ und $R_2$ unabhängig voneinander Substituenten, die unter den erfindungsgemäss angewandten Bedingungen nicht reduziert werden können. Beispielsweise können $R_1$ und $R_2$ Alkyl oder Alkoxy, vorzugsweise mit 1 bis 4 Kohlenstoffatomen, Aryl oder Aryloxy, wie z.B. Phenyl oder Phenoxy, Halogen, insbesondere Chlor oder Brom, Hydroxyl, Carboxyl- oder Sulfogruppen oder Derivate dieser Gruppen wie Ester oder Amide bedeuten.

Die Indices m und n sind unabhängig voneinander 0, 1 oder 2. Vorzugsweise bedeuten sie gleichzeitig 0.

In den Verbindungen der Formel (2), worin $R_1$, $R_2$, m und n die genannten Bedeutungen haben, sind $X_1$ und $X_2$ unabhängig voneinander Wasserstoff, Chlor oder Brom, wobei $X_1$ und $X_2$ nicht gleichzeitig Wasserstoff bedeuten. Vorzugsweise sind in den Verbindungen der Formel (2) $X_1$ und $X_2$ Chlor oder $X_1$ ist Wasserstoff und $X_2$ ist Chlor.

Im erfindungsgemässen Verfahren werden die Verbindungen der Formel (2) in einem wasserfreien, aprotischen, organischen Lösungsmittel vorgelegt. Geeignete Lösungsmittel sind z.B. gegebenenfalls halogenierte Kohlenwasserstoff, Ketone, Aether, Carbonsäureester, Sulfone wie z.B. Sulfolan oder Mischungen dieser Lösungsmittel. Besonders vorteilhaft haben sich Benzol, Toluol, Chlorbenzol, o-Dichlorbenzol, Aceton, Dioxan, Tetrahydrofuran und Sulfolan und Mischungen dieser Lösungsmittel erwiesen, wobei mit o-Dichlorbenzol die besten Ergebnisse erzielt werden.

Zur Durchführung des erfindungsgemässen Verfahrens wird ein Edelmetallkatalysator benötigt. Vorzugsweise kommen Platin, Palladium, Iridium, Rhodium, Osmium oder Ruthenium oder aber auch Nickel in Frage. Mischungen bzw. Legierungen dieser Metalle können ebenfalls eingesetzt werden. Mit Platin und vor allem Palladium werden besonders gute Ergebnisse erreicht.

Es kann von Vorteil sein, dem zu verwendenden Katalysator ein Katalysatorgift beizumischen (partiell zu vergiften), um Nebenreaktionen, insbesondere die Reduktion der mittelständigen Doppelbindung sowie die Ueberreduktion der Formylgruppe zum entsprechenden Alkohol oder gar Kohlenwasserstoff zu vermeiden. Vorzugsweise können zu diesem Zweck Schwefel enthaltende Verbindungen wie z.B. Thioharnstoff, 2-Mercaptobenzthiazol oder Schwefel in Chinolin (vgl. Berichte 54 (1921) 425) verwendet werden.

Der Katalysator wird gewöhnlich auf einen Träger aufgebracht, wobei der Gehalt an Katalysator im allgemeinen 0,5 bis 25 % beträgt. Geeignete Träger sind beispielsweise Aktivkohle, Barium- und Calciumsulfat, Aluminiumoxid und Siliciumdioxid, wobei Aktivkohle und Bariumsulfat bevorzugt sind.

Im allgemeinen werden 5 bis 25 % Katalysator (einschliesslich Träger) bezogen auf zu reduzierendes Substrat im erfindungsgemässen Verfahren eingesetzt.

Die Verwendung eines Säureakzeptors in der Reaktionslösung kann vorteilhaft sein, um den während der Hydrierung entstehenden Halogenwasserstoff zu binden. Halogenwasserstoff hat die nachteilige Eigenschaft, auf die eingesetzten Katalysatoren desaktivierend wirken zu können. Als geeignete Säureakzeptoren kommen beispielsweise Basen wie N,N-Dimethylanilin, N,N-Dimethylacetamid, Aethyldiisopropylamin, 2,6-Dimethylpyridin, Natriumacetat, Magnesiumoxid oder Natriumcarbonat in Frage, wobei N,N-Dimethylanilin, N,N-Dimethylacetamid, Aethyldiisopropylamin und 2,6-Dimethylpyridin besonders geeignet sind. Diese bevorzugten Säureakzeptoren können gleichzeitig auch als Lösungsmittel dienen.

Reaktionstemperatur und- druck können je nach Ausgangsverbindung, Lösungsmittel und Katalysator in weiten Grenzen schwanken. Im allgemeinen strebt man ein Optimum zwischen der für ein bestimmtes System von Reaktanden charakteristischen Kinetik (kurze Reaktionszeit) und Selektivität (hohe Ausbeute an gewünschtum Produkt) an.

Für das erfindungsgemässe Verfahren haben sich Temperaturen von 15 bis 200, insbesondere von 70 bis 140 °C, und Drucke von 1 bis 50, insbesondere von 1 bis 10 bar als günstig erwiesen.

Das erfindungsgemässe Verfahren kann als beendet angesehen werden, wenn eine bestimmte, zuvor berechnete Menge Wasserstoff vom Reaktionsgemisch aufgenommen worden ist. Unter berechneter Menge Wasserstoff soll diejenige Menge Wasserstoff verstanden werden, die theoretisch benötigt wird, um von den Verbindungen der Formel (2), die sowohl eine als auch zwei Säurechloridgruppen enthalten können, zu den Verbindungen der Formel (1) zu gelangen.

Die Reaktionszeit beträgt in der Regel 30 Minuten bis 6 Stunden. Die Aufarbeitung des Reaktionsgemisches erfolgt in an sich üblicher Weise. Nach Entfernen des Katalysators, z.B. durch Filtrieren oder Zentrifugieren, wird das Lösungsmittel entfernt, und der Rohaldehyd durch z.B. Destillation, Umkristallisation oder Bildung von Additionsproduke mit Natriumbisulfit in reiner Form gewonnen.

In einer bevorzugten Ausführungsform des erfindungsgemässen Verfahrens werden die Verbindungen der Formel (2), worin $R_1$, $R_2$, m, n, $X_1$ und $X_2$ die angegebenen Bedeutungen haben, in einem Gemisch von o-Dichlorbenzol und 2,6-Dimethylpyridin in Gegenwart eines Palladiumkatalysators, der mit Schwefel in Chinolin partiell vergiftet ist, bei einer Temperatur von 70 bis 80 °C und bei einem Druck von 1,1 bis 3 bar hydriert, bis eine zuvor berechnete Menge Wasserstoff aufgenommen worden ist.

Durch Reduktion von solchen Verbindungen der Formel (2), worin $X_1$ und $X_2$ Fluor, Chlor oder Brom sind, können auch die neuen Verbindungen der Formel (2) erhalten werden, worin $X_1$ Wasserstoff und $X_2$ Fluor, Chlor oder Brom ist. Die Hydrierung der Ausgangsverbindungen wird unterbrochen, wenn die theoretisch bestimmte Menge Wasserstoff vom Reaktionsgemisch aufgenommen worden ist.

Von diesen neuen Verbindungen kommt der Verbindung der Formel

(3) 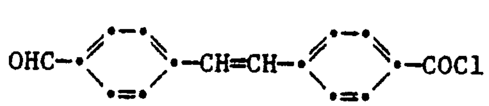

besondere Bedeutung zu.

Sowohl die Herstellung der neuen Verbindungen der Formel (2), die eine Formyl- und eine Säurehalogenidgruppe enthalten, als auch die Verwendung dieser Verbindungen zur Herstellung der Verbindungen Formel (1) geschieht vorzugsweise unter den oben angegebenen Bedingungen.

Die Verbindungen der Formel (1) sind wertvolle Zwischenprodukte z.B. für die Herstellung von optischen Aufhellern.

Beispiel 1: In einem Hydriergefäss (z.B. einer Parr-Niederdruckhydrierbombe) werden 30,5 g (0.1) 4,4'-Stilbendicarbonsäurechlorid, 700 ml o-Dichlorbenzol (über Phosphorpentoxid getrocknet) und 21,9 g 2,6-Dimethylpyridin vorgelegt. Dazu spült man mit 50 ml o-Dichlorbenzol, 6 g Palladiumkatalysator (Bariumsulfatträger mit 5 Gew.-% Palladium) und 75 mg Chinolin-S (hergestellt nach Berichte 54 (1921) 425). Bei 70 °C und 1,1 bar Wasserstoffdruck wird hydriert, bis nach 6 Stunden 0,2 mol Wasserstoff aufgenommen worden sind. Die Hydrierung kommt dann praktisch zum Stillstand.

Danach trennt man den Katalysator und das gebildete Salz durch Filtration von der Reaktionslösung ab und dampft zur Trockne ein. Man erhält so 17 g (72 %) praktisch reinen 4,4'-Stilbendialdehyds mit einem Schmelzpunkt von 169-171 °C. Die Signale in NMR- und IR-Spektren entsprechen der erwarteten Struktur der erhaltenen Verbindung.

Aehnliche Ergebnisse werden erhalten, wenn anstelle des Palladiumkatalysators ein Platinkatalysator eingesetzt wird.

Beispiel 2: Verfährt man wie in Beispiel 1 beschrieben, leitet aber nur die Hälfte der in Beispiel 1 angegebenen Menge Wasserstoff ein und kocht die Reaktionslösung mit 500 ml Aethanol, so kann man dünnschichtchromatographisch 4-Formyl-4'-Stilbencarbonsäureäthylester (Derivat des 4-Formyl-4'-Stilbencarbonsäurechlorids) erhalten.

Beispiel 3: Verfährt man wie in Beispiel 2 beschrieben, engt aber die Reaktionslösung zur Trockne ein, so zeigt ein vom Rückstand aufgenommenes IR-Spektrum die für 4-Formyl-4'-Stilbencarbonsäurechlorid erwarteten Signale.

## Patentansprüche

1. Verfahren zur Herstellung von Verbindungen der Formel

(1) 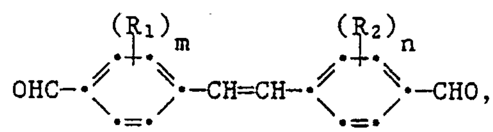

worin $R_1$ und $R_2$ unabhängig voneinander Substituenten sind, die unter den unten definierten Bedingungen nicht reduziert werden können, und m und n unabhängig voneinander 0, 1 oder 2 sind, dadurch gekennzeichnet, dass man Verbindungen der Formel

(2) 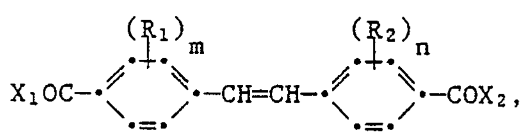

worin $R_1$, $R_2$, m und n die angegebenen Bedeutungen haben und $X_1$ und $X_2$ unabhängig voneinander Wasserstoff, Chlor oder Brom sind, wobei $X_1$ oder $X_2$ nicht gleichzeitig Wasserstoff sind, in einem wasserfreien, inerten, aprotischen, organischen Lösungsmittel in Gegenwart eines Edelmetallkatalysators bei einer Temperatur von 15 bis 200 °C und einem Druck von 1 bis 50 bar so lange hydriert, bis die zuvor berechnete Menge Wasserstoff aufgenommen worden ist, den Katalysator abtrennt und die Verbindung isoliert.

2. Verfahren nach Anspruch 1, dadurch gekennzeichnet, dass als Lösungsmittel gegebenenfalls halogenierte Kohlenwasserstoffe, Ketone, Aether, Carbonsäureester oder Sulfone oder eine Mischung dieser Lösungsmittel verwendet wird.

3. Verfahren nach Anspruch 2, dadurch gekennzeichnet, dass als Lösungsmittel Benzol, Toluol, Chlorbenzol, o-Dichlorbenzol, Aceton, Dioxan, Tetrahydrofuran oder Sulfolan oder eine Mischung dieser Lösungsmittel verwendet wird.

4. Verfahren nach Anspruch 1, dadurch gekennzeichnet, dass als Lösungsmittel o-Dichlorbenzol verwendet wird.

**5.** Verfahren nach Anspruch 1, dadurch gekennzeichnet, dass als Edelmetallkatalysator Platin, Palladium, Nickel, Irdium, Rhodium, Osmium oder Ruthenium oder eine Mischung bzw. Legierung dieser Metalle eingesetzt wird.

**6.** Verfahren nach Anspruch 5, dadurch gekennzeichnet, dass als Edelmetallkatalysator Platin oder Palladium eingesetzt wird.

**7.** Verfahren nach Anspruch 6, dadurch gekennzeichnet, dass als Edelmetallkatalysator Palladium eingesetzt wird.

**8.** Verfahren nach Anspruch 5, dadurch gekennzeichnet, dass der Edelmetallkatalysator partiell vergiftet ist.

**9.** Verfahren nach Anspruch 8, dadurch gekennzeichnet, dass als Katalysatorgift Schwefel in Chinolin, Thioharnstoff oder 2-Mercaptobenzthiazol verwendet wird.

**10.** Verfahren nach Anspruch 1, dadurch gekennzeichnet, dass als Hydrierung in Gegenwart eines Säureakzeptors durchgeführt wird.

**11.** Verfahren nach Anspruch 10, dadurch gekennzeichnet, dass als Säureakzeptor N,N-Dimethylanilin, N,N-Dimethylacetamid, Aethyldiisopropylamin, 2,6-Dimethylpyridin, Natriumacetat, Magnesiumoxid oder Natriumcarbonat verwendet wird.

**12.** Verfahren nach Anspruch 11, dadurch gekennzeichnet, dass als Säureakzeptor N,N-Dimethylanilin, N,N-Dimethylacetamid, Aethyldiisopropylamin oder 2,6-Dimethylpyridin verwendet wird.

**13.** Verfahren nach Anspruch 1, dadurch gekennzeichnet, dass die Säureakzeptoren gemäss Anspruch 11 als Lösungsmittel verwendet werden.

**14.** Verfahren nach Anspruch 1, dadurch gekennzeichnet, dass die Temperatur 70 bis 140 °C beträgt.

**15.** Verfahren nach Anspruch 1, dadurch gekennzeichnet, dass der Druck 1 bis 10 bar beträgt.

**16.** Verfahren nach einem der Ansprüche 1 bis 15, dadurch gekennzeichnet, dass man Verbindungen der Formel (2), worin m und n 0 sind und $X_1$ und $X_2$ unabhängig voneinander Wasserstoff, Chlor oder Brom sind, wobei $X_1$ und $X_2$ nicht gleichzeitig Wasserstoff sind, in einem Gemisch von o-Dichlorbenzol und 2,6-Dimethylpyridin in Gegenwart eines Palladiumkatalysators, der mit Schwefel in Chinolin partiell vergiftet ist, bei einer Temperatur von 70 bis 80 °C und bei einem Druck von 1,1 bis 3 bar hydriert.

**17.** Verbindungen der Formel (2), nach Anspruch 1, worin $R_1$, $R_2$, m und n die angegebenen Bedeutungen haben und $X_1$ Wasserstoff und $X_2$ Chlor oder Brom ist.

**18.** Verbindung nach Anspruch 17 der Formel

(3) 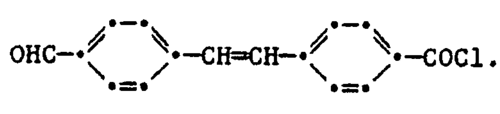

**19.** Verfahren zur Herstellung der Verbindungen nach Anspruch 17, dadurch gekennzeichnet, dass man die Verbindungen der Formel (2), worin $R_1$, $R_2$, m und n die angegebenen Bedeutungen haben und $X_1$ und $X_2$ unabhängig voneinander Chlor oder Brom sind, in einem wasserfreien, inerten, aprotischen, organischen Lösungsmittel in Gegenwart eines Edelmetallkatalysators bei einer Temperatur von 15 bis 200 °C und einem Druck von 1 bis 50 bar so lange hydriert, bis die zuvor berechnete Menge Wasserstoff aufgenommen worden ist, den Katalysator abtrennt und die Verbindung isoliert.

**20.** Verfahren nach Anspruch 19 zur Herstellung der Verbindung der Formel (3), dadurch gekennzeichnet, dass man die Verbindungen der Formel (2), worin m und n 0 sind und $X_1$ und $X_2$ Chlor oder Brom sind, in einem Gemisch von o-Dichlorbenzol und 2,6-Dimethylpyridin in Gegenwart eines Palladiumkatalysators, der mit Schwefel in Chinolin partiell vergiftet ist, bei einer Temperatur von 70 bis 80 °C und bei einem Druck von 1,1 bis 3 bar hydriert.

## Claims

**1.** A process for the preparation of a compound of formula

(1) 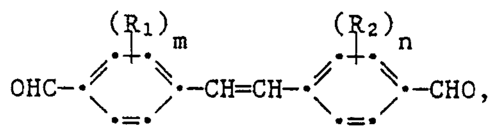

in which $R_1$ and $R_2$ are each independently of the other substituents that cannot be reduced under the reaction conditions defined below, and m and n are each independently of the other 0, 1 or 2, which process comprises hydrogenating a compound of formula

(2) 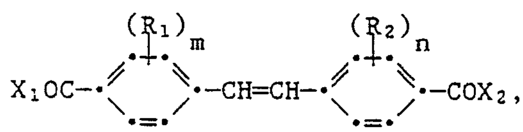

in which $R_1$, $R_2$, m and n are as defined above and $X_1$ and $X_2$ are each independently of the other hydrogen, chlorine or bromine, where $X_1$ and $X_2$ are not simultaneously hydrogen, in an anhydrous, inert, aprotic organic solvent and in the presence of a noble metal catalyst, at a temperature from 15 to 200 °C and under a pressure of 1 to 50 bar, until the absorption of the precalculated amount of hydrogen is complete, then separating the catalyst and isolating the compound.

**2.** A process according to claim 1, wherein the solvent used is a hydrocarbon or halogenated hydrocarbon, a ketone, ether, carboxylic acid ester or sulfone, or a mixture of said solvents.

**3.** A process according to claim 2, wherein the solvent used is benzene, toluene, chlorobenzene, o-dichlorobenzene, acetone, dioxane, tetrahydrofuran or sulfolane, or a mixture of said solvents.

**4.** A process according to claim 1, wherein the solvent used is o-dichlorobenzene.

**5.** A process according to claim 1, wherein the noble metal catalyst used is platinum, palladium, nickel, iridium, rhodium, osmium or ruthenium, or a mixture or alloy of said metals.

**6.** A process according to claim 5, wherein the noble metal catalyst used is platinum or palladium.

**7.** A process according to claim 6, wherein the noble metal catalyst used is palladium.

**8.** A process according to claim 5, wherein the noble metal catalyst is partially poisoned.

**9.** A process according to claim 8, wherein the catalyst poison used is sulfur in quinoline, thiourea or 2-mercaptobenzothiazole.

**10.** A process according to claim 1, wherein the hydrogenation is carried out in the presence of an acid acceptor.

**11.** A process according to claim 10, wherein the acid acceptor used is N,N-dimethylaniline, N,N-dimethylacetamide, ethyldiisopropylamine, 2,6-dimethylpyridine, sodium acetate, magnesium oxide or

sodium carbonate.

**12.** A process according to claim 11, wherein the acid acceptor used is N,N-dimethylaniline, N,N-dimethylacetamide, ethyldiisopropylamine or 2,6-dimethylpyridine.

**13.** A process according to claim 1, wherein the acid acceptors according to claim 11 is used as solvent.

**14.** A process according to claim 1, wherein the temperature is in the range from 70 to 140 °C.

**15.** A process according to claim 1, wherein the pressure is 1 to 10 bar.

**16.** A process according to any one of claims 1 to 15, which comprises hydrogenating a compound of formula (2), in which m and n are 0 and $X_1$ and $X_2$ are each independently of the other hydrogen, chlorine or bromine, where $X_1$ and $X_2$ are not simultaneously hydrogen, in a mixture of o-dichlorobenzene and 2,6-dimethylpyridine in the presence of a palladium catalyst which is partially poisoned with sulfur in quinoline, at a temperature from 70 to 80 °C and under a pressure of 1.1 to 3 bar.

**17.** A compound of formula (2) according to claim 1 in which $R_1$, $R_2$, m and n have the given meanings and $X_1$ is hydrogen and $X_2$ is chlorine or bromine.

**18.** A compound according to claim 17 of formula

(3) 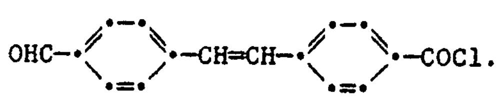

**19.** A process for the preparation of a compound according to claim 17, which comprises hydrogenating a compound of formula (2), in which $R_1$, $R_2$, m and n have the given meanings and $X_1$ and $X_2$ are each independently of the other chlorine or bromine, in an anhydrous, inert, aprotic organic solvent in the presence of a noble metal catalyst, at a temperature from 15 to 200 °C and under a pressure of 1 to 50 bar, until the absorption of the precalculated amount of hydrogen is complete, then separating the catalyst and isolating the compound.

**20.** A process according to claim 19 for the preparation of the compound of formula (3), which comprises hydrogenating a compound of formula (2), in which m and n are 0 and $X_1$ and $X_2$ are chlorine or bromine, in a mixture of o-dichlorobenzene and 2,6-dimethylpyridine in the presence of a palladium catalyst which is partially poisoned with sulfur in quinoline, at a temperature from 70 to 80 °C and under a pressure of 1.1 to 3 bar.

**Revendications**

**1.** Procédé pour la préparation de composés de formule

(1) 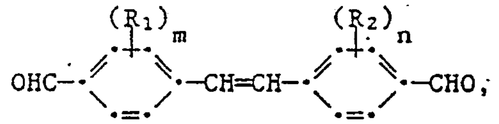

dans laquelle $R_1$ et $R_2$, indépendamment l'un de l'autre, sont des substituants qui ne peuvent pas être réduits dans les conditions définies ci-dessous, et m et n valent, indépendamment l'un de l'autre, 0, 1 ou 2, caractérisé en ce que l'on soumet à une hydrogénation, à une température de 15 à 200 °C et sous une pression de 1 à 50 bars, des composés de formule

(2) 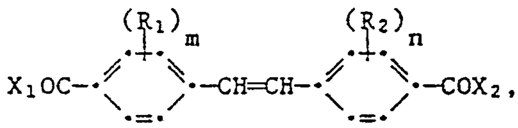

dans laquelle $R_1$, $R_2$, m et n ont les significations données, et $X_1$ et $X_2$ sont, indépendamment l'un de l'autre, un atome d'hydrogène, de chlore ou de brome, $X_1$ ou $X_2$ n'étant pas simultanément des atomes d'hydrogène, dans un solvant organique, aprotique,inerte, anhydre, en présence d'un catalyseur de type métal noble, jusqu'à ce que la quantité d'hydrogène préalablement calculée soit absorbée, on sépare le catalyseur et on isole le composé.

**2.** Procédé selon la revendication 1, caractérisé en ce que l'on utilise, en tant que solvant, des hydrocarbures éventuellement halogénés, des cétones, des éthers, des esters d'acides carboxyliques ou des sulfones, ou un mélange de ces solvants.

**3.** Procédé selon la revendication 2, caractérisé en ce que l'on utilise, en tant que solvant, le benzène, le toluène, le chlorobenzène, l'o-dichlorobenzène, l'acétone, le dioxanne, le tétrahydrofuranne ou le Sulfolane ou un mélange de ces solvants.

**4.** Procédé selon la revendication 1, caractérisé en ce que l'on utilise, en tant que solvant, l'o-dichlorobenzène.

**5.** Procédé selon la revendication 1, caractérisé en ce que l'on utilise, en tant que catalyseur de type métal noble, le platine, le palladium, le nickel, l'iridium, le rhodium, l'osmium ou le ruthénium, ou un mélange ou alliage de ces métaux.

**6.** Procédé selon la revendication 5, caractérisé en ce que l'on utilise, en tant que catalyseur de type métal noble, le platine ou le palladium.

**7.** Procédé selon la revendication 6, caractérisé en ce que l'on utilise, en tant que catalyseur de type métal noble, le palladium.

**8.** Procédé selon la revendication 5, caractérisé en ce que le catalyseur de type métal noble est partiellement empoisonné.

**9.** Procédé selon la revendication 8, caractérisé en ce que l'on utilise, en tant que poison de catalyseur, du soufre dans de la quinoléine, de la thiourée ou du 2-mercaptobenzothiazole.

**10.** Procédé selon la revendication 1, caractérisé en ce l'on effectue l'hydrogénation en présence d'un accepteur d'acide.

**11.** Procédé selon la revendication 10, caractérisé en ce que l'on utilise, en tant qu'accepteur d'acide, la N,N-diméthylaniline, le N,N-diméthylacétamide, l'éthyldiisopropylamine, la 2,6-diméthylpyridine, l'acétate de sodium, l'oxyde de magnésium ou le carbonate de sodium.

**12.** Procédé selon la revendication 11, caractérisé en ce que l'on utilise, en tant qu'accepteur d'acide, la N,N-diméthylaniline, le N,N-diméthylacétamide, l'éthyldiisopropylamine ou la 2,6-diméthylpyridine.

**13.** Procédé selon la revendication 1, caractérisé en ce que les accepteurs d'acide selon la revendication 11 sont utilisés en tant que solvant.

**14.** Procédé selon la revendication 1, caractérisé en ce que la température est de 70 à 140 °C.

**15.** Procédé selon la revendication 1, caractérisé en ce que la pression est de 1 à 10 bars.

**16.** Procédé selon l'une des revendications 1 à 15, caractérisé en ce que l'on soumet à une hydrogénation, à une température de 70 à 80 °C et sous une pression de 1,1 à 3 bars, des composés de formule (2)

dans laquelle m et n valent 0 et $X_1$ et $X_2$ sont indépendamment l'un de l'autre un atome d'hydrogène, de chlore ou de brome, $X_1$ et $X_2$ n'étant Pas en même temps des atomes d'hydrogène, dans un mélange d'o-dichlorobenzène et de 2,6-diméthylpyridine, en présence d'un catalyseur à base de palladium qui est partiellement empoisonné avec du soufre dans de la quinoléine.

**17.** Composés de formule (2) selon la revendication 1, dans lesquels $R_1$, $R_2$, m et n ont les significations données et $X_1$ est un atome d'hydrogène et $X_2$ est un atome de chlore ou de brome.

**18.** Composé selon la revendication 17, de formule

(3) 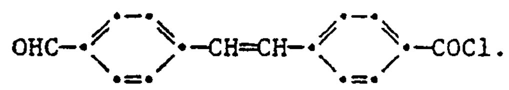

**19.** Procédé pour la préparation des composés selon la revendication 17, caractérisé en ce que l'on soumet à une hydrogénation, à une température de 15 à 200 °C et sous une pression de 1 à 50 bars, les composés de formule (2) dans laquelle $R_1$, $R_2$, m et n ont les significations données, et $X_1$ et $X_2$ sont indépendamment l'un de l'autre un atome de chlore ou de brome, dans un solvant organique, aprotique, inerte, anhydre, en présence d'un catalyseur de type métal noble, jusqu'à ce que la quantité d'hydrogène préalablement calculée soit absorbée, on sépare le catalyseur et on isole le composé.

**20.** Procédé selon la revendication 19, pour la préparation du composé de formule (3), caractérisé en ce que l'on soumet à une hydrogénation, à une température de 70 à 80 °C et sous une pression de 1,1 à 3 bars, les composés de formule (2) dans lesquels m et n valent 0 et $X_1$ et $X_2$ sont des atomes de chlore ou de brome, dans un mélange d'o-dichlorobenzène et de 2,6-diméthylpyridine, en présence d'un catalyseur à base de palladium qui est partiellement empoisonné avec du soufre dans de la quinoléine.